(19) Europäisches Patentamt
European Patent Office
Office européen des brevets

(11) Veröffentlichungsnummer: **0 061 588**
**A1**

# (12) EUROPÄISCHE PATENTANMELDUNG

(21) Anmeldenummer: **82101489.1**

(22) Anmeldetag: **26.02.82**

(51) Int. Cl.$^3$: **C 07 D 215/18**
C 07 D 239/30, C 07 D 213/61

(30) Priorität: **28.03.81 DE 3112415**

(43) Veröffentlichungstag der Anmeldung:
**06.10.82 Patentblatt 82/40**

(84) Benannte Vertragsstaaten:
**AT DE FR GB IT**

(71) Anmelder: **DYNAMIT NOBEL AKTIENGESELLSCHAFT**
**Patentabteilung Postfach 1209**
**D-5210 Troisdorf, Bez. Köln(DE)**

(72) Erfinder: **Hofmann, Volker, Dr.**
**Poststrasse 101**
**D-5210 Troisdorf(DE)**

(72) Erfinder: **Peeters, Hermann, Dr.**
**Porzer Strasse 1**
**D-5216 Niederkassel 3(DE)**

(72) Erfinder: **Steffen, Klaus-Dieter, Dr.**
**Röckelstrasse 36**
**D-5202 Hennef 1(DE)**

(72) Erfinder: **Vogt, Wilhelm, Dr.**
**Kleiststrasse 39**
**D-5000 Köln 40(DE)**

## (54) Verfahren zur Chlorierung von cyclischen Amiden und cyclischen vinylogen Amiden.

(57) Chlorierte Stickstoffheterocyclen, bestehend aus einem oder mehr Ringen, werden durch Chlorierung der entsprechenden cyclischen Amide oder vinylogenen Amide mittels seitenkettenchlorierter Aromaten, die mindestens eine Gruppe $-CCl_3$ enthalten, hergestellt.

EP 0 061 588 A1

Croydon Printing Company Ltd

Troisdorf, den 27.März 1981
OZ: 81 020 ( 4038 )

DYNAMIT NOBEL AKTIENGESELLSCHAFT
Troisdorf, Bez. Köln

# Verfahren zur Chlorierung von cyclischen Amiden und cyclischen vinylogen Amiden

Die vorliegende Erfindung betrifft ein Verfahren zur Herstellung von chlorierten Stickstoffheterocyclen aus cyclischen Amiden durch Umsetzung mit seitenkettenchlorierten Aromaten.

Die Darstellung von chlorierten Stickstoffheterocyclen aus den entsprechenden cyclischen Amiden mit Hilfe von u.a. Phosphorchloriden wie $POCl_3$, $PCl_3$ oder $PCl_5$ ist zur Darstellung von 4,7-Dichlorchinolin in Organic Synthesis Coll. Vol. III, S. 272 beschrieben. Die Ausbeuten sind mit 55-60 % nicht befriedigend. Die sich bildende Phosphorsäure sowie NaCl-haltige Abwässer bereiten im technischen Maßstab Schwierigkeiten. Vorschläge zur Aufarbeitung mit wässriger Salzsäure und Neutralisation benötigen große Mengen Hilfsstoffe, die nach der Reaktion z.B. als Abwasser zu beseitigen sind und der Anforderung einer geringen Belastung der Umwelt nicht entsprechen.

Es bestand daher die Aufgabe, in einer einfachen chemischen Umsetzung die chlorierten Stickstoffheterocyclen in guten Ausbeuten zu synthetisieren und das Verfahren umweltfreundlich, d.h. unter möglichst vollständiger Verwertung aller Stoffe und geringem Verbrauch von Hilfsstoffen zu gestalten.

Gegenstand der Erfindung ist ein Verfahren zur Herstellung von chlorierten Stickstoffheterocyclen der Formeln

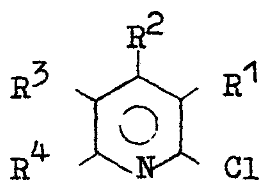

1 a

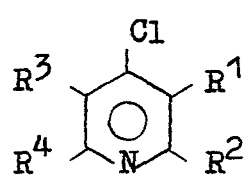

1 b

worin $R^1$, $R^2$, $R^3$ und $R^4$ gleich oder verschieden ist und die Bedeutung Wasserstoff, Cyano, Alkoxy, Nitro, Amino, Mercapto, Alkylmercapto, $-CF_3$, Halogen, Aryl- oder Heteroarylgruppen, Alkylgruppen mit 1 bis 10 C-Atomen, wobei ein oder mehrere Wasserstoffatome der Gruppen $R^1$ bis $R^4$ ihrerseits durch eine der genannten Gruppen $R^1$ bis $R^4$ ersetzt sein können oder zwei benachbarte oder ggf. nicht benachbarte der Reste $R^1$ bis $R^4$ gemeinsam einen carbocyclischen oder heterocyclischen, gegebenenfalls N-haltigen Ring bilden oder einer bzw. mehrere der Reste $R^1$ bis $R^4$ zusammen mit dem zugehörigen Kohlenstoffatom durch Stickstoff ersetzt ist, welches dadurch gekennzeichnet ist, daß ein cyclisches Amid der Formel (2a) oder ein cyclisches vinyloges Amid der Formel (2b)

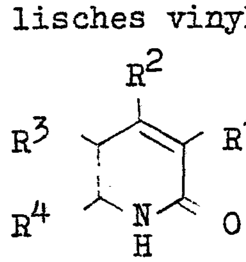

2 a

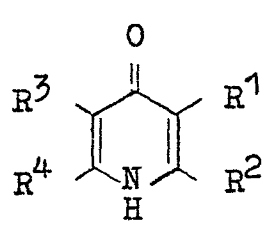

2 b

worin $R^1$, $R^2$, $R^3$ und $R^4$ dasselbe wie oben bedeuten, mit einem seitenkettenchlorierten Aromaten der Formel

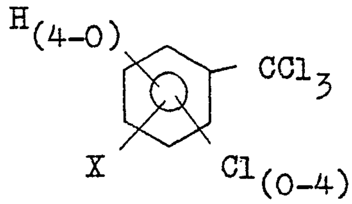

worin X Wasserstoff oder $-CH_{(3-n)}Cl_n$ oder einen bei der Reaktion inerten Substituenten bedeutet und n 0 bis 3 ist, umgesetzt sind.

Als Substituenten $R^1$ bis $R^4$ sind Reste möglich, die sich bei der Reaktion inert verhalten bzw. den Reaktionsablauf nicht stören.
Die Art der Substituenten wird ganz durch die jeweilige Struktur der benötigten heterocyclischen Verbindung bzw. der Struktur der Ausgangsstoffe bestimmt, die sehr vielseitig ist.
Bedarf besteht u.a. an heterocyclischen Verbindungen aus zwei oder mehr Ringen, wobei der zweite Ring bevorzugt ebenfalls ein aromatischer Ring ist. Die Ringe können ein oder zwei Stickstoffatome enthalten. In einer Reihe von Fällen enthalten bereits die Ausgangsstoffe ein oder mehrere Halogensubstituenten, besonders Chlor- oder Trifluormethylsubstituenten.
Als cyclische Amide der Formel 2 a und cyclische vinylogene Amide der Formel 2 b kommen, neben anderen, 4-Hydroxi- bzw. 2-Hydroxichinolin, deren Derivate, besonders Chlorderivate wie die 7-Chlorderivate und 7- oder 8-Trifluormethylderivate und 4-Hydroxipyrimidin und dessen Derivate, wie dessen 2-Phenylderivat in Frage.
Verbindungen, in denen Reste $R^1$ bis $R^4$ zusammen mit dem zugehörigen Kohlenstoffatom durch Stickstoff ersetzt sind, sind u.a. Uracil und 4-Hydroxipyridin.

Das Verfahren wird so durchgeführt, daß das Amid und der seitenkettenchlorierte Aromat, gegebenenfalls in einem inerten Lösungsmittel, suspendiert in Anwesenheit oder Abwesenheit eines Katalysators zusammen aufgeheizt werden und der entstehende Chlorwasserstoff z.B. mit einem Inertgas aus dem Reaktionsgefäß ausgetrieben wird. Der Chlorwasserstoff ist rein und kann absorbiert und wieder verwendet werden.

Nach der Reaktion findet sich außer dem Produkt lediglich das gebildete Säurechlorid vor, sowie Lösungsmittel, Katalysator und der Überschuß des seitenkettenchlorierten Aromaten, soweit diese Stoffe verwendet wurden.

Das Verfahren der Erfindung löst demnach die gestellte Aufgabe in überraschender und vorteilhafter Weise.

Die seitenkettenchlorierten Aromaten enthalten die Gruppe $-CCl_3$, daneben gegebenenfalls eine zweite Gruppe $-CCl_3$ oder $-CHCl_2$ oder/und Chlorsubstituenten im Kern oder weiteren Seitenketten.

Diese Stoffe sind großtechnische Erzeugnisse der Chlorierung von Toluol und der Xylole.

Bevorzugt wird Benzotrichlorid, sowie gegebenenfalls 1,3- und 1,4-Bis-trichlormethylbenzol und 2-Dichlormethylbenzotrichlorid sowie deren Kernsubstitutionsprodukte, die zu Benzoylchlorid, Iso- und Terephthalsäuredichlorid bzw. 3-Chlorphthalid reagieren, die ihrerseits industriell benutzte organische Zwischenprodukt sind.

Das Molverhältnis von Amid zu seitenkettenchloriertem Aromat beträgt 1 : 1 bis 1 : 2,vorzugsweise 1 : 1 bis 1 : 1,25, bei Vorliegen einer Chlor übertragenen Seitengruppe und 1 : 0,5 bis 1 : 1, vorzugsweise 1 : 0,5 bis 1 : 0,625 bei Vorliegen zweier Chlor übertragender Gruppen. Dabei kann sowohl die Trichlormethyl- als auch die Dichlormethylgruppe ein Chlor auf das Amid übertragen.

Die Reaktionstemperatur beträgt 140°C bis 260°C, vorzugsweise 170°C bis 240°C.

Die Reaktion verläuft in Abwesenheit und in Anwesenheit eines die Reaktionspartner suspendierenden Lösungsmittels in gleicher Weise. Die Lösungsmittel müssen chemisch inert und thermisch bei der Reaktionstemperatur stabil sein. Als solche kommen z.B. Diphenyläther, Diphenyl, Wärmeträgeröle verschiedenster Art ( Handelsprodukte Dowtherm®, Marlotherm® und ähnliche Produkte auf Basis von Alkyl- oder Alkylidenaromaten bzw. der entsprechenden kondensierten Aromaten, beispielsweise Gemische von isomeren Triaryldimethanen ) Dichlorbenzole o.ä. in Frage. Die Menge des Lösungsmittels ist nicht kritisch und kann z.B. bis 500 ml pro mol Amid betragen.

Katalysatoren haben nur einen geringen Einfluß auf die Ausbeute, die Reaktionstemperatur und die Reaktionsgeschwindigkeit. Als Katalysatoren geeignet sind insbesondere Lewis-Säuren wie beispielsweise Zinkchlorid, Eisen-(3)-chlorid, Aluminiumchlorid, Titantetrachlorid, Zirkontetrachlorid, Molybdänchlorid oder Molybdänoxid.

Soweit Katalysatoren verwendet werden, eignen sich Mengen von 0,01 bis 5 Molprozent, vorzugsweise von 0,1 bis 2,5 Molprozent.
Der Reaktionsverlauf kann durch Messung der gebildeten HCl verfolgt werden.

Die Aufarbeitung des Reaktionsansatzes kann in verschiedener Weise erfolgen in Abhängigkeit von den Eigenschaften des gebildeten Substrates. Die chlorierten Stickstoff-Heterocyclen können durch Extraktion mit einer wäßrigen Mineralsäure und anschließender Ausfällung durch Basenzugabe gewonnen werden. Vorteilhaft wird das enthaltene

Säurechlorid vor dem Kontakt mit Wasser mit einem Alkohol, vorzugsweise Methanol, zu einem Ester umgesetzt, der als organische Phase von der wäßrigen Lösung abgetrennt und isoliert wird. Der z.B. so einfach gewonnene Benzoesäuremethylester ist ein vielfach benötigtes Produkt. Die vollständige Umsetzung zu diesem Ester, auch von Resten der seitenkettenchlorierten Aromaten, trägt weiter zur Lösung der Aufgabe im Sinne geringerer Belastung der Umwelt bei. Besonders vorteilhaft ist die Verwendung von Alkalialkoholaten bzw. der alkoholischen Lösungen, wodurch zugleich die Neutralisation des Ansatzes erfolgt.

Eine technisch einfache Aufarbeitung, besonders für 4,7-Dichlorchinolin besteht darin, das Reaktionsgemisch mit Alkoholen, vorzugsweise Methanol umzusetzen, mit Alkalialkoholat, vorzugsweise mit einer methanolischen Natriummethylat-Lösung, zu neutralisieren und dann eine fraktionierte Destillation durchzuführen, wobei z.B. zu - erst Methanol, dann Benzoesäuremethylester, darauf 4,7-Dichlorchinolin und schließlich - im Falle der Verwendung von hochsiedenden Lösungsmitteln - dieses abdestilliert wird.
Die Destillation kann diskontinuierlich oder kontinuierlich in mehreren Kolonnen durchgeführt werden. Der organische Destillationsrückstand ist sehr gering.

Da die bei der Chlorierung des Heterocyclus freiwerdende HCl gasförmig entweicht und in Wasser absorbiert wird, wird das zuzusetzende Alkalialkoholat nur zur Neutralisation der HCl aus der Veresterung benötigt.

Die hier synthetisierbaren chlorhaltigen Stickstoffheterocyclen sind Ausgangschemikalien für wertvolle Pharmazeutika wie Chloroquin, Glafenin oder Amodiaquin.

Dr.La/Ra

1 Beispiel 1

45,4 g (0,25 mol) 7-Chlor-4-hydroxichinolin (98,8 Gew.-%) und 51,3 g (0,263 mol) Benzotrichlorid werden unter Stickstoff 2,4 Stunden auf 200°C erhitzt, wobei 0,25 mol HCl freigesetzt werden. Nach dem Abkühlen werden 100 ml 1,1,1-Trichloräthan und 16 g (0,5 mol) Methanol zugesetzt und 2 Stunden bei Raumtemperatur gerührt. Die organische Phase wird mit 150 ml 10 Gew.-%iger Salzsäure extrahiert, anschließend mit Natriumsulfat getrocknet und vom Lösungsmittel befreit. Rückstand: 33,9 g (99,6 % d.Th.) Benzoesäuremethylester. Die wäßrige Lösung wird mit Natronlauge alkalisch gestellt, wobei ein Feststoff ausfällt, der getrocknet wird: 40,0 g (80,8 % d.Th.) 4,7-Dichlorchinolin (Schmp.: 82-84°C).

Beispiel 2

45,4 g (0,25 mol) 7-Chlor-4-hydroxichinolin (98,8 Gew.-%) und 51,3 g (0,263 mol) Benzotrichlorid werden mit 0,25 g (1,3 mmol) Titantetrachlorid 2,0 Stunden auf 200°C erhitzt unter Abspaltung von 0,25 mol HCl. Nach Aufarbeitung wie im Beispiel 1 werden 33,3 g (98,8 % d.Th.) Benzoesäuremethylester und 41,5 g (83,8 % d.Th.) 4,7-Dichlorchinolin erhalten.

Beispiel 3

215,5 g (1,2 mol) 7-Chlor-4-hydroxichinolin und 246,3 g (1,26 mol) Benzotrichlorid werden vorgelegt und unter Stickstoff 2 Stunden auf 200°C erhitzt, wobei 1,2 mol HCl abgespalten werden. Nach dem Abkühlen des Reaktionsansatzes wird unter weiterer Kühlung mit 523 g (1,4 mol) einer 14,5 proz. methanolischen Natriummethylatlösung vorsichtig neutralisiert. Nach Abklingen der exothermen

Reaktion werden nacheinander 403 g (98,7 % d.Th.) Methanol (Kp.: 65°C), 154 g (94,1 % d.Th.) Benzoesäuremethylester ($Kp_{14}$: 80°C und nach Aufheizen auf 85°C 182 g (76,6 % d.Th.) 4,7-Dichlorchinolin ($Kp_{13}$: 145°C) abdestilliert.

Beispiel 5

45,4 g (0,25 mol) 7-Chlor-4-hydroxichinolin (98,8 Gew.%ig) und 51,3 g (0,263 mol) Benzotrichlorid werden mit 1 g (7 mmol) Molybdäntrioxid in 60 ml 1,2-Dichlorbenzol 3 Stunden zum Rückfluß erhitzt unter Abspaltung von 0,22 mol HCl. Nach Aufarbeitung wie im Beispiel 1 wurden 35,1 g (70,9 % d.Th.) 4,7-Dichlorchinolin erhalten.

Beispiel 6

45,4 g (0,25 mol) 7-Chlor-4-hydroxichinolin (98,8 Gew.%ig) und 51,3 g (0,263 mol) Benzotrichlorid werden in 50 ml eines käuflichen Wärmeträgeröles (Marlotherm® der Chemischen Werke Hüls ) 1,75 Stunden auf 200°C erhitzt unter Abspaltung von 0,25 mol HCl. Nach dem Abkühlen werden 16 g (0,5 mol) Methanol und 17,3 g (0,263 mol) Natriummethylat zugegeben und anschließend fraktioniert destilliert. Es werden neben Methanol 29,0 g (85 % d.Th.) Benzoesäuremethylester und 39,4 g ( 79,6 % d.Th.) 4,7-Dichlorchinolin ($Kp._{0,05}$ = 95 - 105°C ) erhalten.
Auch der Wärmeträger kann abdestilliert und bei den folgenden Ansätzen wieder eingesetzt werden.

Beispiel 7

22,7 g (0,015 mol) 7-Chlor-4-hydroxichinolin (98,8 Gew.%ig) und 19,6 g (0,0625 mol) 1,4-Bis-(trichlormethyl-)benzol werden mit 0,125 g (0,8 mmol) Eisen-3-chlorid 1,5 Stunden

auf 200°C erhitzt unter Abspaltung von 0,1 mol HCl. Nach Aufarbeitung wie im Beispiel 1 werden 12,6 g (50,9 % d.Th.) 4,7-Dichlorchinolin erhalten.

Beispiel 8

4,35 g (0,03 mol) 2-Hydroxichinolin und 6,15 g (0,0315 mol) Benzotrichlorid werden 1,5 Stunden auf 200°C erhitzt unter Abspaltung von 0,033 mol HCl. Das Reaktionsgemisch wird nach dem Erkalten mit 10 proz. Salzsäure extrahiert, die wäßrige Lösung mit Natronlauge alkalisch gestellt und diese mit Methylenchlorid extrahiert. Nach dem Trocknen und Abziehen des Lösungsmittels werden 2,0 g (40,8 % d.Th.) 2-Chlorchinolin destilliert.(Kp.: $_{0,05}$ = 80 - 95 °C)

Beispiel 9

17,2 g (0,1 mol) 4-Hydroxi-2-phenylpyrimidin, 20,5 g (0,105 mol) Benzotrichlorid und 1 g (7 mmol) Molybdän-6-oxid werden 1,5 Stunden auf 200°C erhitzt unter Abspaltung von 0,105 mol HCl. Nach Zugabe von 75 ml Wasser wird mit Methylenchlorid extrahiert, die organische Phase mit Natriumsulfat getrocknet, und das Lösungsmittel abgezogen. Nach Umkristallisation aus Ethanol erhält man 14,1 g (74,0 % d.Th.) 4-Chlor-2-phenylpyrimidin.

Dr. La/Ra

## Patentansprüche

1. Verfahren zur Herstellung von chlorierten Stickstoff-heterocyclen der Formeln

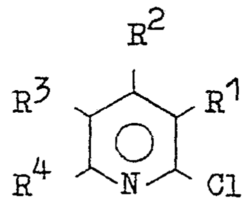

1 a

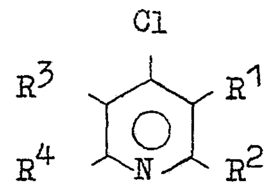

1 b

worin $R^1$, $R^2$, $R^3$ und $R^4$ gleich oder verschieden ist und die Bedeutung Wasserstoff, Cyano, Alkoxy, Nitro, Amino, Mercapto, Alkylmercapto, $-CF_3$, Halogen, Aryl- oder Heteroarylgruppen, Alkylgruppen mit 1 bis 10 C-Atomen, wobei ein oder mehrere Wasserstoffatome der Gruppen $R^1$ bis $R^4$ ihrerseits durch eine der genannten Gruppen $R^1$ bis $R^4$ ersetzt sein können oder zwei benachbarte oder ggf. nicht benachbarte der Reste $R^1$ bis $R^4$ gemeinsam einen carbocylclischen oder heterocyclischen, gegebenenfalls N-haltigen Ring bilden oder einer bzw. mehrere der Reste $R^1$ bis $R^4$ zusammen mit dem zugehörigen Kohlenstoffatom durch Stickstoff ersetzt ist, dadurch gekennzeichnet, daß ein cyclisches Amid der Formel (2a) oder ein cyclisches vinyloges Amid der Formel (2b)

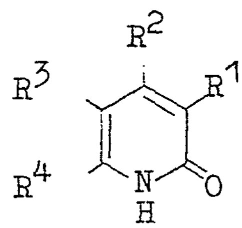

2 a

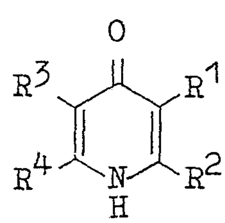

2 b

worin $R^1$, $R^2$, $R^3$ und $R^4$ dasselbe wie oben bedeuten, mit einem seitenkettenchlorierten Aromaten der Formel

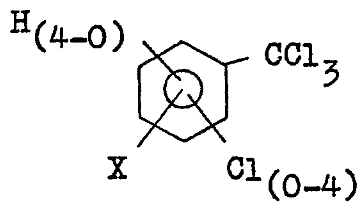

worin X Wasserstoff oder $-CH_{(3-n)}Cl_n$ oder einen bei der Reaktion inerten Substituenten bedeutet und n 0 bis 3 ist, umgesetzt sind.

2. Verfahren nach Anspruch 1, dadurch gekennzeichnet, daß das Molverhältnis der cyclischen Amide zu den seitenkettenchlorierten Aromaten 1 : 1 bis 1 : 2, vorzugsweise 1 : 1 bis 1 : 1,25, bei Vorliegen einer chlorübertragenen Seitengruppe und 1 : 0,5 bis 1 : 1, vorzugsweise 1 : 0,5 bis 1 : 0,625, bei Vorliegen zweier chlorübertragenden Gruppen ist.

3. Verfahren nach einem der Ansprüche 1 oder 2, dadurch gekennzeichnet, daß die Reaktionstemperatur 140 bis 260°C beträgt.

4. Verfahren nach mindestens einem der Ansprüche 1 bis 3, dadurch gekennzeichnet, daß man nach der Reaktion die aus dem Chlorierungsmittel entstandenen Säurechloride verestert und die Ester isoliert.

5. Verfahren nach Anspruch 4, dadurch gekennzeichnet, daß die Veresterung und zugleich die Neutralisation des Ansatzes mit Alkalialkoholaten erfolgt.

Dr.La/Ra

Europäisches Patentamt

# EUROPÄISCHER RECHERCHENBERICHT

0061588

Nummer der Anmeldung

EP 82101489.1

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | DE - A - 1 668 958 (ROUSSEL-UCLAF)<br>* Anspruch 1; Seite 4 *<br>-- | 1 | C 07 D 215/18<br>C 07 D 239/30<br>C 07 D 213/61 |
| A | DE - A1 - 2 814 330 (CASSELLA)<br>* Anspruch 1 *<br>-- | 1 | |
| D,A | ORGANIC SYNTHESES, Collective Volume 3, Annual Volumes 20-29<br>JOHN WILEY & SONS, INC. New York Seiten 272-275<br>---- | 1 | RECHERCHIERTE SACHGEBIETE (Int. Cl.³)<br>C 07 D 215/00<br>C 07 D 239/00<br>C 07 D 213/00 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 23-06-1982 | HOCHHAUSER |